# EUROPÄISCHE PATENTSCHRIFT

(11) **EP 3 265 164 B1**
(45) Veröffentlichungstag und Bekanntmachung des Hinweises auf die Patenterteilung: **10.07.2024**
(21) Anmeldenummer: 16711692.0
(22) Anmeldetag: 23.02.2016
(51) Int. Cl.: A61M 25/10

(54) **VORRICHTUNG ZUR VERLAGERUNG EINES HOHLORGANS EINES PATIENTEN**
DEVICE FOR THE DISPLACEMENT OF A HOLLOW ORGAN OF A PATIENT
DISPOSITIF POUR DÉPLACER UN ORGANE CREUX D'UN PATIENT

(30) Priorität: 05.03.2015 DE 102015103213
(43) Veröffentlichungstag der Anmeldung: 10.01.2018
(73) Patentinhaber: MedFact engineering GmbH, 79540 Lörrach (DE)
(72) Erfinder: VAN HELFTEREN, Alwin, 79379 Müllheim-Feldberg (DE); REINHARDT, Jörg, 79639 Grenzach-Wyhlen (DE)
(74) Vertreter: RACKETTE Patentanwälte PartG mbB
(86) Internationale Anmeldenummer: PCT/IB2016/050954
(87) Internationale Veröffentlichungsnummer: WO 2016/139552

(56) Entgegenhaltungen:
- WO-A1-2012/096885
- WO-A2-2007/046860
- GB-A- 2 370 779
- US-A- 5 395 333
- US-A1- 2008 249 463
- US-A1- 2011 082 488
- US-A1- 2014 277 319

## Beschreibung

Die Erfindung betrifft eine Vorrichtung zur Verlagerung eines Hohlorgans eines Patienten gemäß dem Oberbegriff des Patentanspruchs 1.

Auf der 80. Jahrestagung der Deutschen Gesellschaft für Kardiologie-, Herz- und Kreislaufforschung e.V. in Mannheim vom 23. bis 26. April 2014 wurde von Heiko Lehrmann et al. ein Verfahren vorgestellt, bei dem eine Verlagerung des Ösophagus während einer Pulmonalvenenisolation mittels Cryotechnologie unter Einsatz einer TEE-Sonde (transösophagialen Echokardiographie-Sonde) erfolgt.

In der US 5,170,803 sind eine Vorrichtung und ein Verfahren beschrieben, um einen Ösophagus vorm Herzen weg zu verlagern.

Alle vorbeschriebenen Vorrichtungen haben jedoch den Nachteil, dass die auf den Ösophagus auszuübende Kraft zur Verlagerung durch Druck auf eine verhältnismäßig kleine Oberfläche erfolgt.

Um die für die Verlagerung erforderliche Kraft auf einen Ösophagus zu verteilen, sieht die amerikanische Patentanmeldung US 2011/0082488 A1 einen asymmetrisch aufweitbaren Ballonkatheter vor. Der länglich gestreckte Ballon ist zur Erzeugung einer Asymmetrie mit einem Streifen versehen, der gegenüber dem flexiblen Material des Ballons eine geringere Flexibilität aufweist und dadurch eine einseitige Versteifung herbeiführt, so dass der Ballon sich beim Aufweiten verbiegt und den Ösophagus vom Bereich des linken Atriums eines Herzens wegbiegt.

In der WO 2007/046860 A2 ist eine Vorrichtung mit aufweitbaren Ballons beschrieben, die es gestattet, temporär einen Hohlraum auf Oberflächen des Herzens zu erzeugen. Ein temporärer Hohlraum kann mit dieser Vorrichtung auch auf einem Ösophagus erstellt werden, um diesen von anderen Organen zu entfernen.

Die US 5 395 333 offenbart einen Ballonkatheter mit einer Vielzahl von aufweitbaren Ballons zum Kontaktieren von Blutgefäßwänden.

Der Erfindung, welche im unabhängigen Anspruch 1 sowie den untergeordneten abhängigen Ansprüchen 2 bis 4 definiert ist, liegt die Aufgabe zugrunde, eine Vorrichtung zur Verlagerung eines Ösophagus, zu schaffen, die gut kontrollierbar und mit hoher Präzision hergestellt und eingesetzt werden kann.

Diese Aufgabe wird erfindungsgemäß dadurch gelöst, dass bei einer Vorrichtung der eingangs genannten Art der Katheterschaft drei Durchgänge aufweist, die im Ballonbereich von einem elastischen Ballonschlauch sowie einem Abdecktubus, der über eine distale, eine intermediäre und eine proximale Ausnehmung verfügt, die entlang der Achse des Katheterschaftes abwechselnd in entgegengesetzte Richtungen weisen, umgeben ist, und der in der Nähe der Durchgänge jeweils eine Ausnehmung aufweist, so dass mit Hilfe eines durch den Katheterschaft und die Durchgänge eingebrachten Fluids ein Aufweiten des Ballonschlauchs innerhalb der Ausnehmungen zur Bildung von Ballons erreichbar ist.

Bei einem zweckmäßigen Ausführungsbeispiel sind mehrere sich transversal zum Katheterschaft erstreckende Ballons vorgesehen, die über ein einziges im Katheterschaft vorhandenes Lumen oder, im Falle des erfinderischen Instruments, über individuell zugeordnete gesonderte Lumina unabhängig voneinander durch Einbringen des Füllmediums aufgeweitet werden.

Zweckmäßig ist es, wenn drei Ballons in axialer Richtung voneinander äquidistant beabstandet angeordnet sind. Vorzugsweise sind die Ballons quer zur Längsrichtung in unterschiedliche Richtungen weisend angeordnet.

Für eine gute Steuerung ist es vorteilhaft, wenn die Ballons entlang der Längsachse der Ballonanordnung abwechselnd radial in entgegengesetzte Richtungen weisend angeordnet sind.

Der Abdecktubus weist in der Nähe des Durchgangs eine Ausnehmung auf, so dass mit Hilfe eines durch den Katheterschaft und den Durchgang eingebrachten Füllmediums, beispielsweise Luft oder Wasser, ein Aufweiten des Ballonschlauchs innerhalb der Ausnehmung zur Bildung eines Ballons erreichbar ist, so dass die angestrebte Verlagerung durch Ändern des verwendeten Drucks des Fluids mit hoher Genauigkeit erfolgen kann.

Eine einfache mechanische Konstruktion ergibt sich, dadurch dass der Abdecktubus über eine distale, eine intermediäre und eine proximale Ausnehmung verfügt. Diese Ausnehmungen sind entlang der Achse des Katheterschaftes abwechselnd in entgegengesetzte Richtungen weisend angeordnet.

Wenn eine gesonderte Ansteuerung der Ballons für eine besonders gute Kontrolle der Verlagerung gewünscht ist, ist es zweckmäßig, wenn der Katheterschaft drei Lumina aufweist, die über jeweils eine gesonderte Speiseleitung mit einer Spritze oder einer Pumpe verbunden sind, um die Dilatation der einzelnen Ballons so zu steuern, dass sich die optimale Krümmung des Hohlorgans, insbesondere eines Ösophagus, ergibt.

Die Ballonanordnung ist von einem äußeren elastischen Ballonschlauch umgeben, so dass diese einer leichteren Reinigung zugänglich ist und sich ein verbesserter optischer Eindruck ergibt.

Nachfolgend werden Ausführungsbeispiele der Erfindung anhand der Zeichnung näher erläutert.

Es zeigen:
- Fig. 1: ein Ausführungsbeispiel der Erfindung in einer perspektivischen schematischen Gesamtansicht,
- Fig. 2: eine vergrößerte schematische Ansicht einer Ballonanordnung gemäß einem Ausführungsbeispiel,

- Fig. 3: die Ballonanordnung gemäß Fig. 2 in einer Seitenansicht teilweise geschnitten,
- Fig. 4: ein Ausführungsbeispiel der Erfindung, bei dem abweichend von dem in Fig. 2 dargestellten Ausführungsbeispiel eine getrennte Dilatation jeden Ballons möglich ist,
- Fig. 5: eine Ansicht auf die Ballonanordnung in Richtung der durch die Ballons aufgespannten Ebene, und
- Fig. 6: einen Schnitt entlang der Linie A-A in Fig. 5 zur Veranschaulichung der Ballonbildung durch Aufweiten eines Ballonschlauchs durch eine Ausnehmung in einem Abdecktubus beim Einpressen eines Fluids über einen Durchgang in der Wandung des Katheterschaftes.

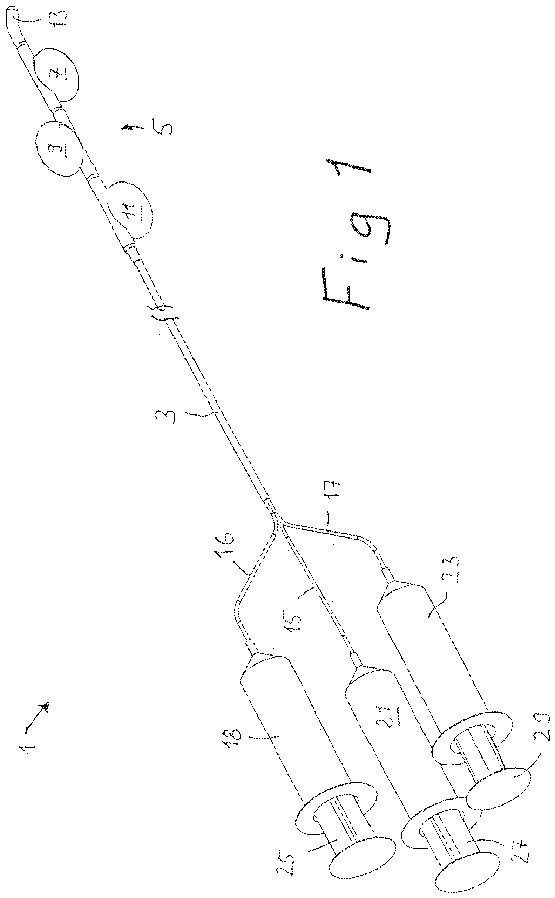

In Fig. 1 erkennt man in perspektivischer Ansicht einen Ballonkatheter 1 mit einem Katheterschaft 3, der aus zeichnerischen Gründen verkürzt und unterbrochen dargestellt ist, und eine Ballonanordnung 5. Die Ballonanordnung 5 umfasst einen distalen Ballon 7, einen intermediären Ballon 9 und einen proximalen Ballon 11. Die seitlich aus dem Katheterschaft 3 herausragenden Ballons 7, 9 und 11 sind entlang der Längsachse der Ballonanordnung 5 äquidistant voneinander angeordnet, wobei in der in Fig. 1 dargestellten Position sich der distale Ballon 7 und der proximale Ballon 11 nach unten und der intermediäre Ballon 9 sich in die entgegengesetzte Richtung nach oben transversal zum Katheterschaft 3 erstrecken. Der distale Ballon 7 befindet sich in der Nähe des distalen Endes 13 des Ballonkatheters 1, welches zur erleichterten Einführung über die Nase oder den Mund zum Ösophagus eines Patienten vorzugsweise etwas gekrümmt sein kann. Die Krümmung 13 zeigt vorzugsweise in die gleiche Richtung wie die beiden parallel orientierten Ballone 7 und 11 um eine anatomisch korrekte Positionierung zu erleichtern.

Bei dem in Fig. 1 dargestellten ersten Ausführungsbeispiel der Erfindung sind dem distalen Ballon 7, dem intermediären Ballon 9 zum Wegdrücken des Ösophagus vom Herzen und dem proximalen Ballon 11 jeweils gesonderte Lumina im Katheterschaft 3 zugeordnet. Die drei Lumina des Katheterschafts 3 sind über den Ballons 7, 9 und 11 jeweils zugeordneten und entsprechend markierten Speiseleitungen 16, 15 und 17 mit Spritzen 18, 21 und 23 verbunden. Die Spritzen 18, 21, 23 mit einem Volumen von 60 bis 120 ml gestatten es, als Füllmedium ein Fluid, beispielsweise Luft oder eine Flüssigkeit, in die jeweils zugeordneten Ballons 7, 9 und 11 zu befördern, um diese auf einen Ballondurchmesser von etwa 10 bis 60 mm aufzuweiten.

Bei einem zweckmäßigen Ausführungsbeispiel sind die Speiseleitungen 13, 16 und 17 jeweils über in der Zeichnung nicht dargestellte Absperrventile (zum Beispiel Luer-Lock System) mit den Spritzen 18, 21, 23 verbunden, die nach dem Aufweiten der Ballons 7, 9 und 10 verschließbar sind, um die Expansion der Ballons 7, 9 und 11 aufrechtzuerhalten. Zum Kollabieren der Ballons werden die Absperrventile geöffnet und die Stempel 25, 27 und 29 herausgedrückt oder herausgezogen.

Anstelle der Verwendung von Spritzen 18, 21, 23 ist es auch möglich, andere Mittel, wie Pumpen, zu verwenden, um die Dilatation der Ballons 7, 9 und 11 zu steuern.

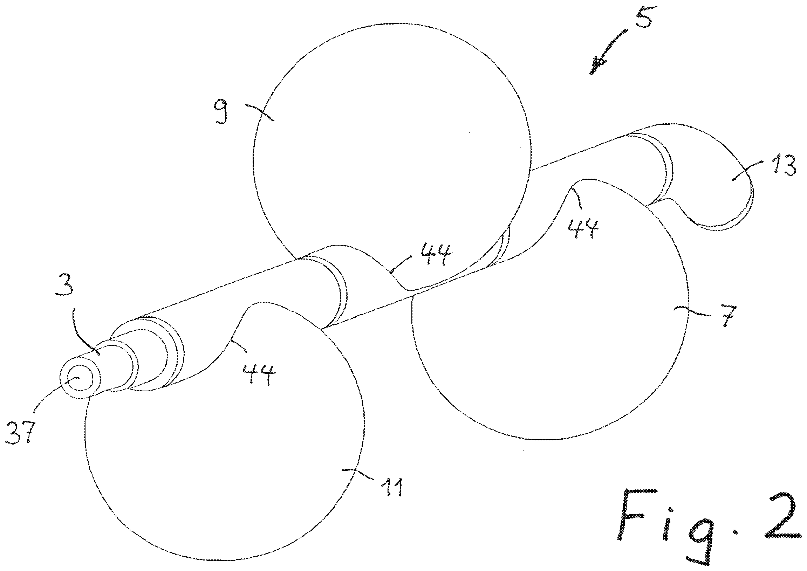

Fig. 2 zeigt ein Ausführungsbeispiel für eine Ballonanordnung 5, bei der der Katheterschaft 3 über ein einziges Lumen 37 verfügt, über das die drei vollständig expandierten Ballons 7, 9 und 11 mit einer einzigen in der Zeichnung nicht dargestellten Spritze verbunden und daher mit deren Hilfe gemeinsam aufweitbar sind.

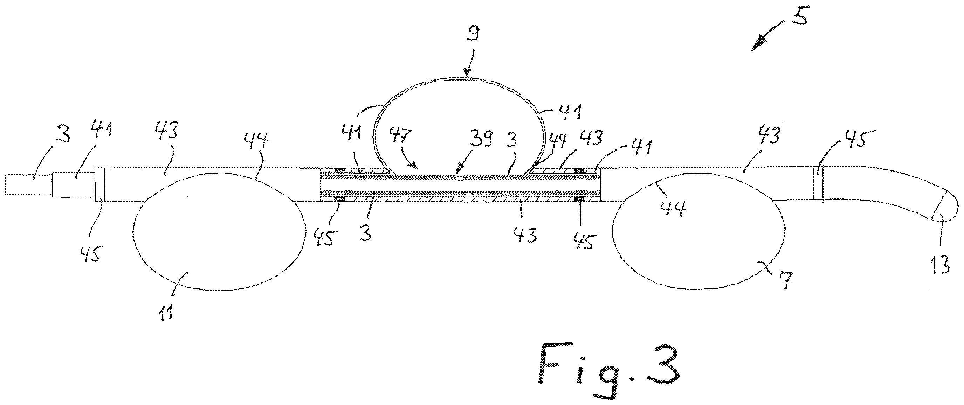

Der Aufbau der in Fig. 2 perspektivisch dargestellten Ballonanordnung 5 ist in der Fig. 3 teilweise geschnitten dargestellt. Der Katheterschaft 3 ist im Bereich der Ballons 7, 9 und 11 jeweils mit einem Durchgang 39 versehen. Beim Einpressen eines Füllmediums mit Hilfe einer Spritze mit einem Volumen zwischen 50 ml und 500 ml gelangt dieses über den Katheterschaft 3 und den Durchgang 39 in das Innere des Ballons 9, wobei die Menge des mit Hilfe einer Spritze oder einer sonstigen Pumpe eingebrachten Füllmediums und die jeweiligen Druckverhältnisse festlegen, wie weit eine Ballonaufweitung stattfindet.

Vorzugsweise bilden sich die Ballons 7, 9 und 11 durch Aufweiten eines sich in Längsrichtung der Ballonanordnung 5 um den Katheterschaft 3 erstreckenden Ballonschlauches 41. Der Ballonschlauch 41 ist dazu, wie in Fig. 3 veranschaulicht, von einem Abdecktubus 43 umgeben, in dem mindestens eine Ausnehmung 47 vorgesehen ist, die ein Aufweiten des Ballonschlauches 41 nur im Bereich der Ausnehmung 47 gestattet.

Der Abdecktubus 43 beziehungsweise die Segmente des Abdecktubus 43 sind zur Bildung der Ballons 7, 9, 11 jeweils mit entlang dem Katheterschaft im Abstand voneinander angeordneten Ausnehmungen 47 versehen, die sich über die vorgesehene Ballonlänge in axialer Richtung und in Umfangsrichtung über etwa die Hälfte des Umfangs mit einer gekrümmten Berandungslinie 44 so erstrecken, dass beim Einbringen des Fluids mit einem Druck durch einen der Durchgänge 39 ein Ausbeulen des Ballonschlauchs 41 ähnlich wie bei einer Hernie erfolgt, da die durch den Abdecktubus 43 gebildete Wandung stabiler ausgebildet ist als der elastische Ballonschlauch 41, der zum Beispiel aus Silikon, Latex, PUR, Chronoprene oder C-Flex besteht.

Der Abdecktubus 43 kann einstückig oder mehrstückig ausgebildet sein, wobei zwischen einzelnen Segmenten des Abdecktubus 43 Ringe 45 vorgesehen sein können, die zur Markierung unter einer Röntgenstrahlung vorgesehen sein können, oder auch eine Funktion zum abdichtenden Andrücken des Ballonschlauchs 41 auf den Katheterschaft 3 übernehmen können. Zur Abdichtung der einzelnen Ballons 7, 9 und 11 kann es zweckmäßig sein, den Abdecktubus 43 sowie den Ballonschlauch 41 zum Beispiel durch Verkleben der Bereiche des Ballonschlauchs 41 zwischen den Stellen, an denen sich Ballons bilden sollen, abzudichten. Auch ein Umwickeln der abzudichtenden Bereiche entlang dem Katheterschaft 3 ist möglich, um eine Abdichtung zu erreichen.

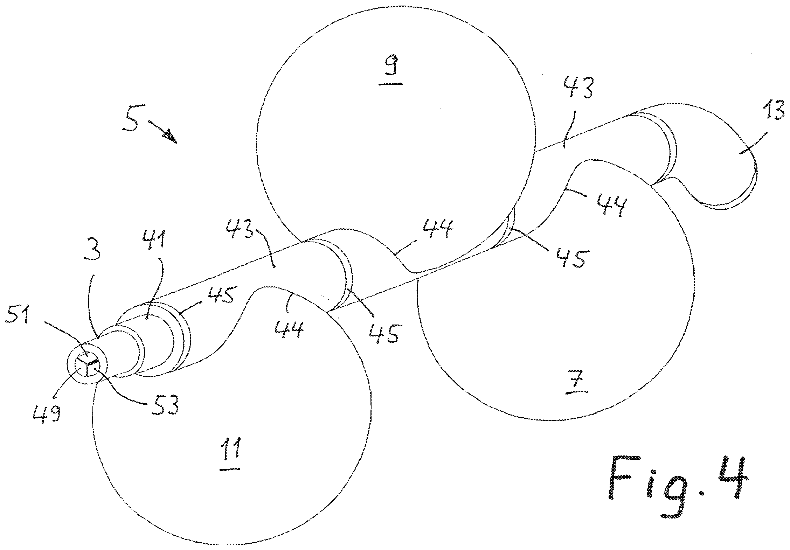

Bei dem Ausführungsbeispiel gemäß Fig. 2 und 3 verfügt der Katheterschaft 3 über ein einziges Lumen, während bei dem anhand Fig. 1 beschriebenen Ausführungsbeispiel der Erfindung der Katheterschaft 3, wie in Fig. 4 veranschaulicht ist, drei voneinander getrennte Lumina 49, 51 und 53 aufweist. Jedes dieser Lumina 49, 51, 53 mündet über gesonderte Durchgänge 39 in nur einen der Ballons 7, 9, 11, so dass diese Ballons 7, 9, 11 jeweils unabhängig voneinander aufgeweitet werden können, um die Andruckkräfte den jeweiligen Bedingungen in einem Hohlorgan, insbesondere einem Ösophagus, gut anpassen zu können.

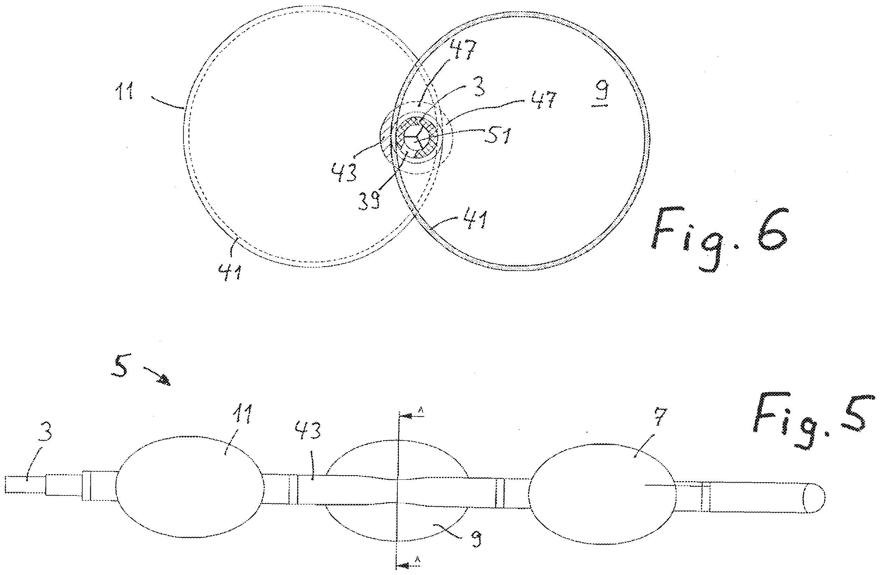

Fig. 5 veranschaulicht eine Draufsicht auf die Ballonanordnung 5 nach dem Aufweiten der Ballons 7, 9 und 11. Zur Verdeutlichung zeigt Fig. 6 einen Schnitt durch den Ballon 9 entlang der Linie A-A in Fig. 5. Das Aufweiten des intermediären Ballons 9 erfolgt über das Einbringen eines Fluids durch das Lumen 51 des Katheterschafts 3 und den Durchgang 39. Der Ballonschlauch 41 hat nach dem Aufweiten zum Ballon 9 in etwa die in Fig. 6 schematisch veranschaulichte Form und ragt durch die Ausnehmung 47 im Abdecktubus 43 heraus, während außerhalb der Ausnehmung 47 der Abdecktubus 43 so dimensioniert ist, dass der Ballonschlauch 41 an einem Aufweiten gehindert ist.

Zweckmäßig ist es, wenn alle Teile der Ballonanordnung 5 außer denjenigen, die zur Markierung dienen, aus für Röntgenstrahlen transparentem Kunststoff bestehen, der biokompatibel ist und die erforderlichen Eigenschaften bezüglich der Elastizität, Flexibilität und Verformbarkeit hat. Dies bedeutet, dass der Ballonschlauch 41 aus einem für Ballons geeigneten leicht ausdehnbaren, aber festem Material besteht, wobei das Material des Abdecktubus 43 bei ausreichender Flexibilität dem Druck standhält, durch den die Ballons 7, 9 und 11 zum Aufweiten gebracht werden sollen.

Bei einem in der Zeichnung nicht dargestellten Ausführungsbeispiel der Erfindung ist die Ballonanordnung 5 von einem zweiten äußeren elastischen Ballonschlauch umgeben, der von den einzelnen Ballons 7, 9, 11 an mehreren Stellen asymmetrisch aufgeweitet werden kann, so dass die Außenhaut der Ballonanordnung überall ohne scharfe Kanten ballonartig abgerundet verläuft, so dass sich insbesondere eine leichtere Reinigung und ein besserer optischer Eindruck ergibt.

## Patentansprüche

1. Vorrichtung (1) zur Verlagerung des Ösophagus eines Patienten während einer Katheterablation bei Herzrhythmusstörungen zur Vermeidung ösophagialer Verletzungen, mit einer im Ösophagus asymmetrisch aufweitbaren Ballonanordnung (5, 7, 9, 11) zum Ausüben eines Verlagerungsdrucks auf die Innenseite des Ösophagus und mit einem mit der aufweitbaren Ballonanordnung (5, 7, 9, 11) verbundenen Katheterschaft (3), über den unter Verwendung eines Füllmediums die asymmetrische Aufweitung der Ballonanordnung (5, 7, 9, 11) steuerbar ist, wobei die aufweitbare Ballonanordnung (5, 7, 9, 11) drei sich transversal zum Katheterschaft (3) erstreckende Ballons (7, 9, 11) aufweist, die entlang der Längsachse der Ballonanordnung (5) abwechselnd radial in entgegengesetzte Richtungen weisend angeordnet sind und über gesonderte Lumina (49, 51, 53) des Katheterschaftes (3) unabhängig voneinander durch Einbringen des Füllmediums aufweitbar sind, **dadurch gekennzeichnet, dass** der Katheterschaft (3) drei Durchgänge (39) aufweist, die im Ballonbereich von einem elastischen Ballonschlauch (41) sowie einem Abdecktubus (43), der über eine distale, eine intermediäre und eine proximale Ausnehmung (47) verfügt, die entlang der Achse des Katheterschaftes (3) abwechselnd in entgegengesetzte Richtungen weisen, umgeben sind, und wobei der Abdecktubus in der Nähe der Durchgänge (39) jeweils eine Ausnehmung (47) aufweist, so dass mit Hilfe eines durch den Katheterschaft (3) und die Durchgänge (39) eingebrachten Fluids ein Aufweiten des Ballonschlauchs (41) innerhalb der Ausnehmungen (47) zur Bildung von Ballons (7, 9, 11) erreichbar ist.

2. Vorrichtung nach Anspruch 1, **dadurch gekennzeichnet, dass** die Ballons (7, 9, 11) in axialer Richtung äquidistant voneinander beabstandet angeordnet sind.

3. Vorrichtung nach Anspruch 1 oder 2, **dadurch gekennzeichnet, dass** der Katheterschaft (3) drei Lumina (49, 51, 53) aufweist, die jeweils über eine Speiseleitung (13, 15, 17) an eine Spritze (18, 21, 23) anschließbar sind, und die in jeweils einen von drei Durchgängen (39) münden, welche jeweils einem sich quer zum Katheterschaft (3) erstreckenden Ballon (7, 9, 11) zugeordnet sind.

4. Vorrichtung nach einem der vorstehenden Ansprüche 1 bis 3, **dadurch gekennzeichnet, dass** das distale Ende (13) des Katheterschaftes (3) gekrümmt ausgebildet ist, wobei die Krümmungsrichtung in die Richtung weist, in der die zwei Ballons (7, 11) weisen.

## Claims

1. Device (1) for displacing the oesophagus of a patient during a catheter ablation for cardiac arrhythmia in order to prevent oesophageal injury, having a balloon arrangement (5, 7, 9, 11) asymmetrically expandable in the oesophagus in order to exert displacement pressure on the inside of the oesophagus and having a catheter shaft (3) connected to the expandable balloon arrangement (5, 7, 9, 11), by which the asymmetric expansion of the balloon arrangement (5, 7, 9, 11) can be controlled with the use of a filling medium, wherein the expandable balloon arrangement (5, 7, 9, 11) has three balloons (7, 9, 11), which extend transversely to the catheter shaft (3) and which are arranged along the longitudinal axis of the balloon arrangement (5) pointing alternately radially in opposite directions and are expandable independently of each other by introduction of the filling medium through separate lumens (49, 51, 53) in the catheter shaft (3), **characterised in that** the catheter shaft (3) has three bores (39), which are enclosed in the area of the balloon by an elastic balloon tube (41) as well as a covering tube (43), which has a distal, an intermediary and a proximal recess (47), which point alternately in opposite directions along the axis of the catheter shaft (3), and wherein the covering tube in the proximity of the bores (39) has a recess (47), so that by means of a fluid introduced through the catheter shaft (3) and the bores (39) an expansion of the balloon tube (41) within the recesses (47) can be achieved for the formation of balloons (7, 9, 11).

2. Device according to Claim 1, **characterised in that** the balloons (7, 9, 11) are arranged equidistantly spaced from each other in an axial direction.

3. Device according to Claim 1 or 2, **characterised in that** the catheter shaft (3) has three lumens (49, 51, 53), which are each connectable to a syringe (18, 21, 23) via a supply line (13, 15, 17) and which each open into one of three bores (39), which are each assigned to a balloon (7, 9, 11), which extends transversely to the catheter shaft (3).

4. Device according to one of the preceding Claims 1 to 3, **characterised in that** the distal end (13) of the catheter shaft (3) is designed curved, wherein the direction of curvature points in the direction in which the two balloons (7, 11) point.

## Revendications

1. Dispositif (1) pour déplacer l'oesophage d'un patient pendant une ablation par cathéter en cas de troubles du rythme cardiaque afin d'éviter des lésions oesophagiennes, avec un ensemble de ballonnets (5, 7, 9, 11) dilatables de manière asymétrique dans l'oesophage pour exercer une pression de déplacement sur le côté intérieur de l'oesophage et avec une tige de cathéter (3) reliée à l'ensemble de ballonnets (5, 7, 9, 11) dilatables par l'intermédiaire de laquelle la dilatation asymétrique de l'ensemble de ballonnets (5, 7, 9, 11) peut être commandée en utilisant un milieu de remplissage, l'ensemble de ballonnets (5, 7, 9, 11) dilatables comportant trois ballonnets (7, 9, 11) s'étendant transversalement à la tige de cathéter (3), qui sont disposés le long de l'axe longitudinal de l'ensemble de ballonnets (5) en étant orientés en alternance radialement dans des directions opposées et qui sont dilatables indépendamment les uns des autres par l'intermédiaire de lumières séparées (49, 51, 53) de la tige de cathéter (3) en introduisant le milieu de remplissage, **caractérisé en ce que** la tige de cathéter (3) comporte trois passages (39) qui sont entourés dans la zone du ballonnet par un tube de ballonnet élastique (41) ainsi que par un tube de recouvrement (43) qui dispose d'un évidement distal, d'un évidement intermédiaire et d'un évidement proximal (47), qui sont orientés alternativement dans des directions opposées le long de l'axe de la tige de cathéter (3), et dans lequel le tube de recouvrement comporte un évidement (47) à proximité de chacun des passages (39), de sorte qu'à l'aide d'un fluide introduit à travers la tige de cathéter (3) et les passages (39), il est possible d'obtenir une dilatation du tube de ballonnet (41) à l'intérieur des évidements (47) pour former des ballonnets (7, 9, 11).

2. Dispositif selon la revendication 1, **caractérisé en ce que** les ballonnets (7, 9, 11) sont disposés à équidistance les uns des autres dans la direction axiale.

3. Dispositif selon la revendication 1 ou 2, **caractérisé en ce que** la tige de cathéter (3) comporte trois lumières (49, 51, 53) qui peuvent être raccordées chacune à une seringue (18, 21, 23) par l'intermédiaire d'une conduite d'alimentation (13, 15, 17) et qui débouchent chacune dans l'un de trois passages (39) qui sont associés chacun à un ballonnet (7, 9, 11) s'étendant transversalement à la tige de cathéter (3).

4. Dispositif selon l'une des revendications 1 à 3 précédentes, **caractérisé en ce que** l'extrémité distale (13) de la tige de cathéter (3) est incurvée, la direction de courbure étant orientée dans la direction dans laquelle les deux ballonnets (7, 11) sont orientés.
